# EUROPEAN PATENT SPECIFICATION

(11) **EP 3 983 059 B1**
(45) Date of publication and mention of the grant of the patent: **05.08.2026**
(21) Application number: 20825732.9
(22) Date of filing: 17.06.2020
(51) Int. Cl.: A61N 1/36, A61B 5/24, A61N 1/00, A61N 1/32, A61N 1/04

(54) **EAR DEVICE INVOLVING NERVE STIMULATION**
OHRVORRICHTUNG MIT NERVENSTIMULATION
DISPOSITIF AUDITIF FAISANT APPEL À LA STIMULATION NERVEUSE

(30) Priority: 17.06.2019 US 201962862446 P
(43) Date of publication of application: 20.04.2022
(73) Proprietor: Evren Technologies, Inc., Newberry, FL 32869 (US)
(72) Inventor: KAROW, Blythe, Newberry, FL 32669 (US); EULIANO, Neil, Gainesville, FL 32608 (US)
(74) Representative: AWA Sweden AB
(86) International application number: PCT/US2020/038262
(87) International publication number: WO 2020/257367

(56) References cited:
- WO-A1-2017/115368
- WO-A1-2018/039458
- WO-A1-2018/071630
- WO-A1-2019/005774
- US-A1- 2003 195 588
- US-A1- 2003 195 588
- US-A1- 2008 021 520
- US-A1- 2013 150 653
- US-A1- 2016 279 021
- US-A1- 2017 143 971
- US-A1- 2017 311 097
- US-A1- 2017 368 329
- US-A1- 2018 133 504
- US-A1- 2018 193 651
- US-A1- 2019 001 129
- US-B2- 7 386 347

## Description

### FIELD OF INVENTION

The present invention relates generally to stimulation transfer apparatuses for use in electrically stimulating medical devices.

### BACKGROUND

Nerve stimulation is relatively known in the art, particularly stimulation of the autonomic nervous system. The autonomic nervous system controls physiological activities of the body and the imbalance of autonomic tone is related to many diseases and conditions. Nerve stimulation, particularly vagus nerve, trigeminal, etc. stimulation, has been proposed to treat many conditions, including, without limit, post-traumatic stress disorder (PTSD), acute stress disorder, traumatic brain injury (TBI), epilepsy, mental illness or disorder, depression, anxiety, sleep disorders, eating disorders, obesity, anorexia, obsessive compulsive disorder (OCD), substance abuse, bipolar disorder, schizophrenia, autism spectrum disorders (ASD), acute stress disorder, gastrointestinal motility, gastrointestinal tract disorders, inflammatory conditions, postpartum depression, hypertension, Mild Cognitive Impairment, incomplete stroke, Partial cord injury, coma, ADHD, insomnia, pain, and epilepsy.

In the treatment of the various disorders, it has been shown that regular stimulation sessions, including chronic stimulation of the autonomic nervous system, including the vagus nerve, trigeminal nerve, etc., may provide for a more effective therapeutic effect. However, devices for nerve stimulation tend to be large and require an operator to deliver and monitor the stimulation to the patient, thus there remains an unmet need for a portable or wearable autonomic nerve system stimulation device which requires only the user to fit to themselves and operate for treatment.

It is further appreciated that with autonomic nerve system stimulation, sympathetic, as well as parasympathetic, activation/inhibition occurs. Much autonomic nerve system stimulation is performed "open loop" where a predetermined degree of stimulation is applied for a predetermined period of time, without regard for the effect that the stimulation is actually having on a patient. "Closed-loop" stimulation is a form of neuromodulation that provides stimulation only when necessary and includes a feedback mechanism through monitoring one or more biological responses obtained from a physiologic sensor and adjusting the stimulation based on the biological response. With the exception of certain breathing systems which monitor CO2 to adjust breathing (thus indirectly affecting the autonomic nervous system), no portable device exists which includes an incorporated means or device for biological monitoring of the patient, thus there remains an unmet need. Furthermore, while "closed-loop" stimulation systems certainly have their advantages, including the ability to optimize therapy, avoid habituation, and minimize side effects, "open-loop" stimulation still may be desired in certain treatment regimens, thus there remains an unmet need for a portable autonomic nerve system stimulation device that may operate either as a "open-loop" or "closed-loop" stimulation system.

Finally, while autonomic stimulation may be effective alone, it is appreciated that paired therapies with stimulation for certain conditions, provide better patient response, recovery and improve overall effectiveness of the treatment in patient's recovery. Thus there remains an unmet need for a portable autonomic nerve system stimulation device that may operate as an "open-loop" or "closed-loop" stimulation system along with a paired therapy for a disease or condition.

US-A 1-2019/001129 discloses a peripheral nerve stimulator to stimulate a peripheral nerve to treat essential tremor and other movement disorders, as well as overactive bladder, cardiac dysfunction and neurotransmitter dysfunction is provided. The peripheral nerve stimulator can be a noninvasive surface stimulator to provide multi-modal optimized therapy. Stimulation can be vibrational, electromechanical, thermal, radiant, electrical, magnetic, electromagnetic, light, mechanical, chemical, thermal, ultrasonic, radiofrequency (RF), acoustic, infrared, ultraviolet, x-ray, and/or microwave. Stimulation can be delivered using an open loop system and/or a closed loop system with feedback. Stimulation can be to one site or multiple sites.

WO-A1 2018/039458 discloses systems and methods for the treatment and prevention of cardiac dysrhythmias and/or hypertension, and more specifically to systems and methods of treating cardiac dysrhythmias, including atrial fibrillation, as well as hypertension through noninvasive peripheral nerve stimulation.

### SUMMARY OF INVENTION

The present invention provides for a portable autonomic nerve system stimulation device that may operate as an "open-loop" or "closed-loop" stimulation system.

One aspect of the invention provides for a portable autonomic nerve system stimulation device which may be worn in the ear, or about the head, of a patient which includes a stimulation circuit for providing a stimulation signal, one or more stimulation transfer apparatii for applying the stimulation, one or more controllers for controlling the stimulation, and one or more biological signal monitoring apparatus for monitoring a patients biological inputs.

Another aspect of the invention provides for a switching circuit, which allows for the inventive device to be switched between modes of treatment, including, without limit, "open-loop", "closed-loop", and "sleep mode".

Another aspect of the invention provides for a switching circuit, which allows for the inventive device to be switched from "open-loop" to "closed-loop" or to allow for a setting in closed-loop operation that effectively remains on at all times, mimicking "open-loop."

Another aspect of the invention is for the incorporation of a communication device for pairing the portable autonomic nerve system stimulation device to a computing system, such as a computer or mobile device, for assisting in providing a nerve stimulation therapy or paired treatment regimen. Where used, certain aspects of the invention allow for remote monitoring and programming of one or more user's the portable autonomic nerve system stimulation device by the user or one or more third parties, including without limit medical personnel or care givers.

This summary is provided to introduce a selection of concepts in a simplified form that are further described below in the Detailed Description. The foregoing has outlined some of the pertinent objects of the invention. These objects should be construed to be merely illustrative of some of the more prominent features and applications of the intended invention. Many other beneficial results can be attained by applying the disclosed invention in a different manner or modifying the invention within the scope of the disclosure. Accordingly, other objects and a fuller understanding of the invention may be had by referring to the summary of the invention and the detailed description of the preferred embodiment in addition to the scope of the invention defined by the claims taken in conjunction with the accompanying drawings.

### BRIEF DESCRIPTION OF DRAWINGS

Examples illustrative of embodiments of the disclosure are described below with reference to figures attached hereto. In the figures, identical structures, elements or parts that appear in more than one figure are generally labeled with the same numeral in all the figures in which they appear. Dimensions of components and features shown in the figures are generally chosen for convenience and clarity of presentation and are not necessarily shown to scale. Many of the figures presented are in the form of schematic illustrations and, as such, certain elements may be drawn greatly simplified or not-to-scale, for illustrative clarity. The figures are not intended to be production drawings. The figures (Figs.) are listed below.

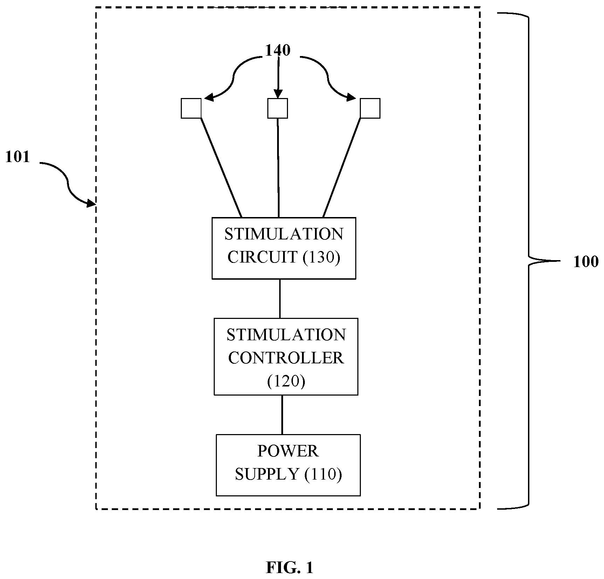

**FIG. 1** provides an illustration of at least one embodiment of the inventive ear device **100** illustrating an optional housing **101,** power supply **110,** stimulation controller **120,** stimulation circuit **130** and one or more stimulation apparatus (or electrodes) **140.**

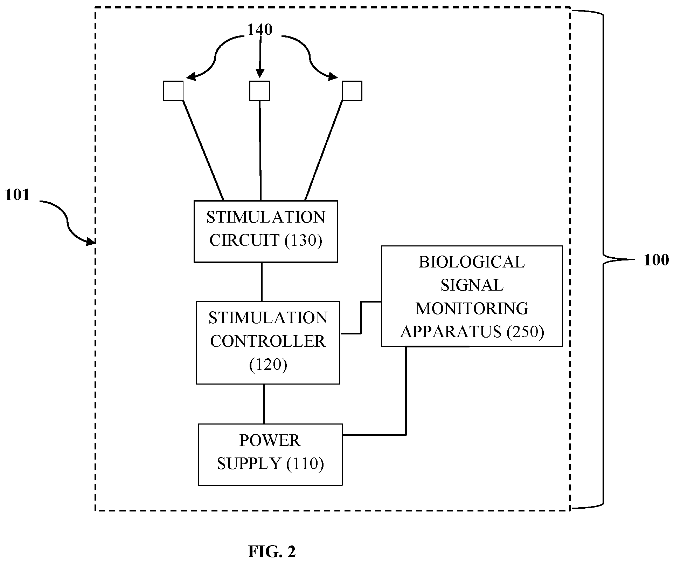

**FIG. 2** provides an illustration of at least one embodiment of the inventive ear device **100** illustrating an optional housing **101,** power supply **110,** stimulation controller **120,** stimulation circuit **130,** one or more stimulation apparatus (or electrodes) **140,** and one or more biological signal monitoring apparatus **250.**

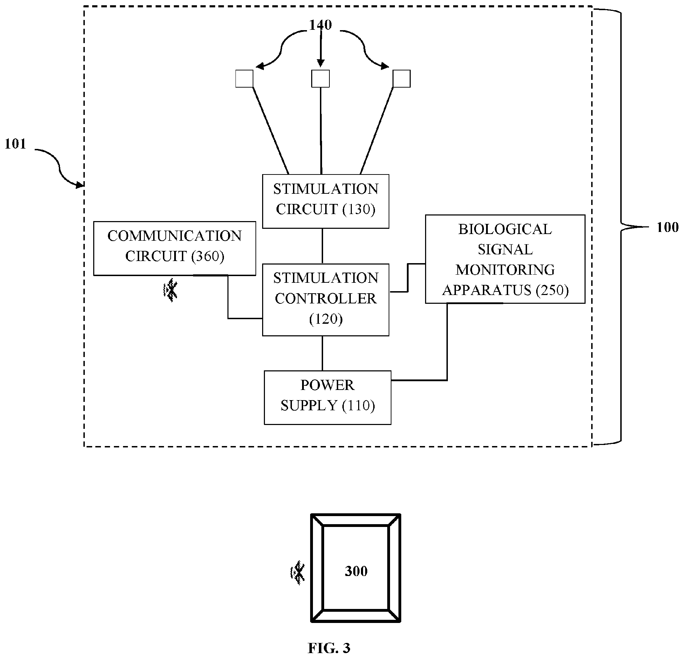

**FIG. 3** provides an illustration of at least one embodiment of the inventive ear device **100** illustrating an optional housing **101,** power supply **110,** stimulation controller **120,** stimulation circuit **130,** one or more stimulation apparatus (or electrodes) **140,** one or more biological signal monitoring apparatus **250,** and a communication circuit **360** for communication with an external processing device such as a mobile device or computer. It should be appreciate that although illustrated separately, the communication circuit may be included as part of the stimulation circuit **130,** stimulation controller **120** or biological signal monitoring apparatus **250.**

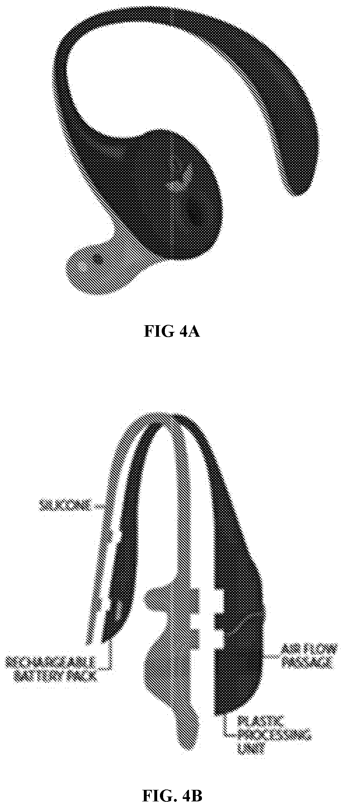

**FIG. 4A** and **FIG. 4B** provides for a perspective view of one embodiment of the inventive ear device **100,** configured as a headset.

It should be clear that the description of the embodiments and attached Figures set forth in this specification serves only for a better understanding of the invention, without limiting its scope. It should also be clear that a person skilled in the art, after reading the present specification could make adjustments or amendments to the attached Figures and above described embodiments that would still be covered by the present invention.

### DETAILED DESCRIPTION

The following detailed description is merely exemplary in nature and is in no way intended to limit the scope of the invention, its application, or uses, which may vary. The invention is described with relation to the non-limiting definitions and terminology included herein. These definitions and terminology are not designed to function as a limitation on the scope or practice of the invention, but are presented for illustrative and descriptive purposes only.

The present invention provides for a portable ear device for delivering autonomic nerve stimulation. The device includes at least one housing, at least one stimulation circuit, at least one biological signal monitoring apparatus and sensor, at least one controller, and at least one power supply. The inventive device provides for a portable autonomic nerve system stimulation device that may operate as a "open-loop" or "closed-loop" stimulation system and includes in some embodiments a paired therapy for a disease or condition

In some embodiments, the housing is intended to fit about or within an ear and intended to contain, at least, the stimulation circuit, stimulation transfer apparatii for applying the stimulation to the patient, and at least one biological signal monitoring apparatus. In at least one embodiment, the housing is intended to be inserted into at least a portion of the pinna of the human ear

The housing is defined by an outer surface having an inner volume which is capable of holding, at least, the stimulation circuit, with one or more stimulation transfer apparatii attached to its outer surface, such that when worn the stimulation transfer apparatii are in a position on the patient to provide nerve stimulation to the autonomic nerve.

In at least one embodiment, one or more of the stimulation transfer apparatii is one or more electrodes. In at least one embodiment the one or more electrodes consist of conductive rubber, silicone, or other flexible conductive material. In such embodiments, the use of the conductive rubber, silicone, or other flexible conductive material increases the contact surface area for providing stimulation and further provides more comfort. Additionally, use of such material extends the expected life span of the electrodes. In at least one embodiment the one or more electrodes consist of flexible hydrogel electrodes. In at least one embodiment the one or more electrodes consist of silver chloride electrodes or sticky electrodes. It should be understood that electrodes may be used with a layer of hydrogel.

In at least one embodiment, the electrodes are metal electrodes with protruding bumps or dimples. Such electrodes may be flat or curved depending on the position where used, or the application in which the electrodes are used. Without being bound to any particular theory, the protrusions and/or shape is intended to increase the contact surface area and create localized areas of increased contact pressure in order to decrease impedance without requiring higher overall contact pressure. In such embodiments, the size and depth of the dimples will dictate the local contract pressure while the size of the overall metal electrode and the device design will dictate the total contact pressure. In combination or in the alternative, such dimples or protrusions may be used with other embodiments of the electrodes described herein, including, without limit the aforementioned conductive rubber or silicone electrodes.

In yet another embodiment of suitable electrodes, the electrode consists of a tight grid of spring-loaded pins which are intended to contact the surface of the skin at a set pressure. The intent of the spring pressure is to have the pins conform to the contact surface regardless of ear geometry. The pins in this embodiment may be made from any suitable conductive material including, without limit, metal, conductive rubber, silicone, or combinations thereof.

In at least one embodiment, the aforementioned pins are combined with compressible material such as rubber or silicone where the pins are embedded in, or backed by, the flexible or compressible material such as rubber or silicone to generate a similar spring-load effect without needing individually spring loaded pins.

In at least one embodiment, the electrodes consist of a dry, or mostly dry, conductive material that conducts electricity via both electrical conductivity and ionic conductivity.

It should further be appreciated that multiple electrode types may be used in a single device or in different devices used for treating different indications. It should be further appreciated that any combination of electrodes described herein, any combination of electrodes known in the art, or any combination of electrodes described herein or known in the art may be used.

In at least one embodiment, the stimulation applied through the stimulation transfer apparatii provides for the application of stimulation generated by the stimulation circuit to be applied to the vagus or trigeminal nerve.

The stimulation circuit is intended to provide the generation and delivery of a stimulation to said one or more stimulation transfer apparatus. In some embodiments the stimulation circuit is located in the inner volume of the housing such that the device can be portable and without requiring additional wires. It is appreciated, that in some embodiments that in order to generate a larger stimulus, an external device may need to be implemented, and nothing herein is intended to prevent the incorporation of an external stimulation circuit with associated connection to the stimulation circuit described herein. It is appreciated that stimulation may come in many forms. In at least one embodiment, the stimulation is electrical stimulation, a vibration transient, a sound transient, or combinations thereof. In at least one embodiment, the stimulation circuit includes a pulse generator.

The at least one biological signal monitoring apparatus is intended to be used for monitoring a biological state of a user wearing the device. In some embodiments, the biological signal monitoring apparatus is located in the inner volume of the housing. Without intending to limit the invention, suitable biological signal monitoring apparatii include a pulse plethysmography device, Photoplethysmography (PPG) device, Pulse Oximeter (PulseOx) device, galvanic skin response (GSR) device, electroencephalogram (EEG) device, Electrocardiogram (ECG) device, Electromyography (EMG) device, or combinations thereof. In at least one embodiment, other sensors can be implemented alone or in combination with the aforementioned biological signal monitoring apparatus. Such additional sensors include an accelerometer, gyroscope, digital compass, or combinations thereof.

Use of multiple sensors allows for the automation of certain treatment regimen. In at least one embodiment, a sensor input from a patient or subject determines when to start stimulation. In such embodiments, the software package is used to correlate sensor data with these inputs and can automatically start stimulation. For example, using such embodiment allows for a patient to provide a movement or motion to automate the commencement of a stimulation session without having to initiate through a user-interface.

In at least one embodiment, the biological signal monitoring apparatus may be used to measure for bradycardia or tachycardia in a user.

The at least one controller is intended for adjusting the stimulation of the stimulation circuit. This adjustment may be in response to a biological signal monitoring apparatus, a user input, an associated software program, a pre-programmed stimulation delivery method, or combinations thereof. The at least one controller is in communication with said stimulation circuit and biological signal monitoring apparatus. In at least one embodiment the communication is electrical communication or wireless communication, or combinations thereof. It should be appreciated that the controller may be external or internal to the housing. In at least one embodiment, the controller is internal to the housing. In some embodiments, the controller is a computing device having at least one processor and at least one software package, that when executed by the processor, the software package performs the steps of providing a signal to initiate stimulation from the stimulation circuit. In some embodiments, the software allows for user defined/controlled stimulation frequency and intensity, and optionally for the monitoring the at least one biological signal monitoring apparatus and adjusting one or more electrical characteristics of the stimulation based on the response from the biological signal monitoring apparatus. It should be further appreciated that the controller may control the stimulation at the stimulation transfer apparatus individually or collectively. In at least one embodiment each stimulation transfer apparatus is controlled individually. In at least one embodiment each stimulation transfer apparatus is controlled collectively.

In at least one embodiment, the software is used to receive a Photoplethysmography (PPG) signal from the patient prior to or upon the initiation of stimulation. The PPG data is then used to calculate R-R intervals then used to calculate the RSA (respiratory sinus arrhythmia) of the subject wearing the portable ear device. The calculated RSA is then used to turn on or off stimulation, or adjust (increase/decrease) the amplitude, pulse width, frequency, or waveform of stimulation for the subject based on a user defined or programmed RSA. In at least one embodiment, the R-R intervals are filtered for integrity. The filtering is accomplished by comparing the measured R-R to previous intervals. The filtering may also be accomplished in some embodiments by determining previous R-R intervals and filtering the R-R intervals based on future intervals or a combination of both past and future intervals. In at least one embodiment, the RSA is calculated on a set time window based on a high frequent filter.

In at least one embodiment, the integrity of the RSA values is assessed both by looking at the other recent (both past and future) RSA values and by looking at the R-R intervals used. In at least one embodiment, an average of RSA values is determined to get an average RSA over a set time span. In at least one embodiment, the on/off of the stimulation, or adjustment (increase/decrease) of the amplitude, pulse width, frequency, or waveform of stimulation takes into account heart rate and movement based on accelerometer. This on off limit accounting for heart rate and movement based on accelerometer is based on population norms, value averages for the user, or a clinically meaningful number for the particular condition being treated. This on off limit accounting for heart rate and movement based on accelerometer also varies based on postural position. In at least one embodiment, the stimulation parameters, intensity, or on/off duration are changed based on identifiable features measured in the RSA, heart rate, pulse oximeter, or accelerometer.

It should be appreciated that embodiments may include other factors when determining the application of stimulus, or stimulates intensity.

In at least one embodiment, demographic data is used to match to historical data of similar patients to determine thresholds for stimulation, or determine entirely different waveforms of programmed modes. In at least one embodiment, current conditions for determining thresholds for stimulation are used. In at least one embodiment a plurality of sensors are implemented and cross correlated for stimulating patient. As a non-limiting example monitoring a patients activity using an accelerometer and cross-correlating such that stimulation is only applied when the subject is stressed, but to not provide stimulation when the subject is exercising.

In at least one embodiment the R-R intervals and/or RSA values are filtered by automatically removing outliers by first removing beats with high/low inter-beat interval, then inter-beat intervals (IBIs) more than a certain percentage (20% for example) above/below local average, then removing those more than a certain percent above the local standard deviation. In at least one embodiment a removed outlying data point is replaced with the average of a number of adjacent data points.

It should be appreciated that where an external device is used, such as an external controller as described above, embodiments of the inventive device may include one or more communication circuits for communicating and receiving data and/or external commands from one or more external devices, wherein said external devices are at least one mobile device, computer, computing device, cellular device, or combinations thereof. In at least one embodiment communication circuit communicates with one or more external devices through Bluetooth, radio frequency, cellular, Wi-Fi, or combinations thereof.

In at least one embodiment of the present invention, the communication circuit allows for remote monitoring or remote programming of the electrical stimulation of said device. In at least one embodiment remote monitoring allows for a secondary person to view the biosignal readings or usage of said device. In at least one embodiment remote programming provides for the ability to send new programs to the user by one or more of the user, a secondary person, the device manufacturer, or combinations thereof. In at least one embodiment the remote programming provides for the ability for a clinician to adjust the stimulation for the patient in real-time. It should further be appreciated that the communication circuit may be included as part of the stimulation circuit, stimulation controller or biological signal monitoring apparatus.

The at least one power supply is intended for providing power to the at least one controller, at least one biological signal monitoring apparatus, or at least one stimulation circuit. It is appreciated that each of these components may have separate or common power supplies. It should be further appreciated that a power supply may be external or internal to the housing. Many power supplies are known in the art. Without intending to limit the availability of power supplies used in the present invention, suitable power supplies include at least one of a rechargeable battery, a disposable battery, an electrical connection to a constant source of electrical power, or combinations thereof.

In at least one embodiment, the inventive device further includes a switching circuit for selectively removing communication from the biological signal monitoring apparatus. In at least one embodiment the switching circuit allows for using communication from the biological signal monitoring apparatus to adjust stimulation settings. In some embodiments, the switching circuit allows to selectively make the device open-loop or closed loop as to a user's preference, or as may be required under a certain treatment regimen. In at least one embodiment the switching circuit allows the inventive device to be placed into sleep-mode. Sleep-mode may be set for a user going to sleep, thus adjusting stimulation parameters, or to temporarily suspend the stimulation features for purposes of taking off the device or power saving. In some embodiments multiple modes are preprogrammed. In at least one embodiment, modes of operation may be programmed by the user or clinician.

In at least one embodiment, if the switching circuit allows for communication from the biological signal monitoring apparatus to the at least one controller, the at least one controller adjusts the stimulation signal based on the biological state received by said biological signal monitoring apparatus of said user. In at least one embodiment, where the stimulation is electrical stimulation, the electrical stimulation is controlled the controller by adjusting the electrical characteristics of the electrical stimulation, wherein said electrical characteristic is the amperage, pulse width, pulse frequency, duty cycle, waveform, or combinations thereof.

In at least one embodiment, the switching circuit provides for a sleep mode in order to allow for limiting the duration of a stimulation sessions. In at least one embodiment, when sleep mode is selected, stimulation is performed that reduced in intensity or frequency at a predetermined amount of time until no stimulation is applied at the predetermined amount of time.

In at least one embodiment, when sleep mode is selected, stimulation is performed that reduced in intensity or frequency at a predetermined amount of time then is switched to closed loop and only provides stimulation based on the signals received by the biological signal monitoring apparatus.

In at least one embodiment, when sleep mode is selected, stimulation is performed that reduced in intensity or frequency at a predetermined amount of time, and then begins stimulation upon detection of the user REM cycle. The user REM cycle may be detected automatically, or may be determined through the implementation of one or more methods for automatically detecting a user's REM cycle. Many methods are known in the art for detecting a user's REM cycle, and nothing herein is intended to limit such detection methods. In at least one embodiment, a biosignal is monitored throughout a user's sleep cycle without stimulating for one or more sleep cycles to determine the presence of a user's REM cycle.

In at least one embodiment the device further includes "exercise mode" which changes the thresholds for application of stimulation, and, where additional biometric sensors are used, cross-compares data to prevent stimulation resulting from exercise but allowing stimulation for the treated condition if occurring during exercise.

In at least one embodiment, the modes are switched automatically based on physiologic feedback.

In some embodiments the device further includes a sound pass-through apparatus which allows external sound to pass through the ear device. In some embodiments this is accomplished by a microphone and speaker or a bone conducting device. The net effect of the pass-through apparatus is to avoid muffling or obstructing sound when the device is worn in normal environments.

One aspect of the invention is to have paired therapy in use with the device. In embodiments incorporating a paired therapy, stimulation is provided to one or more patients using one or more embodiments of the inventive device while conducting one or more therapies related to the condition, injury or disease state of the patient during said application of said stimulation. Without intending to limit this invention, condition, injury or disease states which would benefit from such combination include, without limit, post-traumatic stress disorder (PTSD), acute stress disorder, amnestic mild cognitive impairment, traumatic brain injury (TBI), incomplete spinal cord injury recovery, epilepsy, mental illness or disorder, depression, anxiety, sleep disorders, eating disorders, obsessive compulsive disorder (OCD), substance abuse, bipolar disorder, schizophrenia, autism spectrum disorders (ASD), acute stress disorder, gastrointestinal motility, gastrointestinal tract disorders, postpartum depression, hypertension, Mild Cognitive Impairment, incomplete stroke, Partial cord injury, coma, ADHD, insomnia, pain, and epilepsy, insomnia, pain, various inflammatory disorders, or combinations thereof. Without intending to limit this invention, the therapy regimen using the inventive device could further be paired with other therapies including various forms of physical therapy, cognitive training and learning exercises, Exposure Therapy (CBT, CPT, EMDR, Prolonged Exposure Therapy) and pharmacologic therapies (SSRIs, other drugs).

In at least one embodiment, the setup of the apparatus includes settings related to sex, age, heart conditions (like arrhythmias), and activities of the subject or patient.

Finally, it should be appreciated that where use of a portable ear device for delivering nerve stimulation is used, certain embodiments may require the calibration of the stimulation to provide the effective stimulation for the patient. This may be required to account for daily threshold changes in the patient as a result of chemical or physiological changes, or for use of the device with different patients, or use of the device for different therapy regimens. Where required, the calibration process includes incrementing a stimulation intensity from a sub-perception level until said user initially identifies receipt of said stimulation (paresthesia), then continuing to increment said stimulation from initial paresthesia until the patient notices and/or indicates discomfort from the stimulation, and setting said stimulation intensity to be at a level of 50% - 90% of said stimulation intensity between paresthesia and said stimulation intensity where discomfort was indicated. It should be appreciated that in order to perform the foregoing, that the stimulation parameters would need to be monitored at each respective point of the calibration cycle in order to calculate the calibrated intensity level and associated range.

In one embodiment the calibration process includes setting parameters based on just the user telling indicating when they first feel the stimulation. In at least one embodiment, the calibration process includes random variations of amplitude or other waveform adjustments to the stimulation after a user indicates the stimulation is set. In at least one embodiment the calibration process includes finding what stimulation inputs cause a user to be uncomfortable such that the controller may be set not to exceed such values.

### EXAMPLES

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EXAMPLE 1

A portable ear device for delivering nerve stimulation is provided. The device includes at least one stimulation circuit for the generation and delivery of a stimulation which is connected to one or more stimulation transfer apparatus. The device further includes at least one biological signal monitoring apparatus for monitoring a biological state of a user wearing the device, at least one controller in electrical communication with the stimulation circuit and at least one power supply for providing power to the at least one controller, at least one biological signal monitoring apparatus, and at least one stimulation circuit.

The stimulation transfer apparatus is connected the concha and cymbaconcha of the ear of a subject. The biological signal monitoring apparatus is a PPG (pulse plethysmograph) connected to monitor the ear concha of a subject. The patient or a therapy practitioner may set the stimulation circuit to multiple stimulation modes based on length and intensity of stimulation. The stimulation circuit may also be adjusted to provide the selected stimulation mode for specific periods of time. The controller in electrical communication with the stimulation circuit includes the use of software which is programmed to perform the steps of monitoring R-R intervals in heart rate of a subject and calculates the RSA (respiratory sinus arrhythmia) from those intervals.

### EXAMPLE 2

The portable ear device for delivering nerve stimulation is provided and as provided and connected in Example 1. In this example, the software uses the calculated RSA to initiate and terminate stimulation from the stimulation circuit based on the RSA calculated form the subject.

### EXAMPLE 3

The portable ear device for delivering nerve stimulation is provided and connected as described in Example 1 or Example 2, but the device further includes at least one housing having an outer surface shaped to be inserted into at least a portion of a human ear, where the housing defines an inner volume for holding one or more of the stimulation circuit, biological signal monitoring apparatus, stimulation controller, the power supply or combinations thereof. The housing of the portable ear device of this example further includes one or more stimulation transfer apparatus on its outer surface.

### EXAMPLE 4

The portable ear device for delivering nerve stimulation is provided and connected as described in Example 1, Example 2, or Example 3. One the application of stimulation is initiated, the software implemented first measures PPG from the ear, then calculates R-R intervals based on the PPG data. The R-R intervals are then filtered based on past measurements, to ensure integrity of the received data. After the R-R intervals are filtered, the RSA is calculated on a set time window based on a high frequency filter. The stimulation then is terminated once the measured RSA reaches the predetermined or manually input RSA value. It should be appreciated that the RSA value may differ from patient to patient based on skin conductivity, sensitivity, and condition being treated.

### EXAMPLE 5

The portable ear device for delivering nerve stimulation is provided and connected as described in Example 1, Example 2, Example 3, or Example 4 except that the . biological signal monitoring apparatus is a Pulse Oximeter (PulseOx) device.

### EXAMPLE 6

The portable ear device for delivering nerve stimulation is provided and connected as described in Example 1, Example 2, Example 3, or Example 4 except that the . biological signal monitoring apparatus is a galvanic skin response (GSR) device.

### Other Embodiments

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the described embodiments in any way. Rather, the foregoing detailed description will provide those skilled in the art with a convenient road map for implementing the exemplary embodiment or exemplary embodiments. It should be understood that various changes can be made in the function and arrangement of elements without departing from the scope as set forth in the appended claims and the legal equivalents thereof.

## Claims

1. A portable ear device (100) for delivering a closed-loop nerve stimulation to a skin surface, the device comprising:
one or more stimulation transfer apparatus (140) for transferring an electrical stimulation to said skin surface from at least one stimulation circuit;
at least one stimulation circuit (130) for the generation and delivery of a stimulation to one or more stimulation transfer apparatus, said at least one stimulation circuit (130) electrically connected to said one or more stimulation transfer apparatus;
a first biological signal monitoring apparatus (250), said first biological signal monitoring apparatus (250) being at least one galvanic skin response (GSR) detector for measuring conductance of the skin surface;
one or more communication circuits (360) for communicating and receiving data and/or external commands from one or more external devices to at least one controller provide on demand electrical stimulation from said one or more external devices;
at least one controller (120) in electrical communication with said at least one stimulation circuit (130), one or more communication circuits (360), and said first biological signal monitoring apparatus (250), said controller (120) configured to provide a stimulation signal to the stimulation circuit (130) based on the measured conductance of the galvanic skin response detector;
wherein said controller (120) is configured to provide a stimulation intensity to the stimulation circuit (130) at a level of 50% - 90% of the stimulation intensity between paresthesia and a predetermined stimulation intensity known to cause discomfort to a user at said skin surface upon said GSR detector detecting skin conductance within a predetermined conductance range;
wherein said predetermined stimulation intensity known to cause discomfort is pre-calibrated for a particular user via an initial calibration routine, and stored in memory accessible to the controller (120); and
wherein said controller (120) automatically adjusts the stimulation intensity in real-time based on fluctuations in GSR readings, such that the stimulation remains within a user-specific comfort window.

2. The device of claim 1 further comprising at least one second biological signal monitoring apparatus for monitoring a biological state of a user wearing the device

3. The device of claim 2 wherein said at least one second biological signal monitoring apparatus is a pulse plethysmography device, Photoplethysmography (PPG) device, Pulse Oximeter (PulseOx) device, galvanic skin response (GSR) device, electroencephalogram (EEG) device, Electrocardiogram (ECG) device, Electromyography (EMG) device, or combinations thereof.

4. The device of any of claims 1-3 further comprising at least one housing having an outer surface shaped to be inserted into at least a portion of a human ear, said housing defining an inner volume, and said housing having one or more of said stimulation transfer apparatus on its outer surface.

5. The device of claim 4 wherein said inner volume of said housing contains one or more of the power supply, stimulation circuit, stimulation controller, first or second biological signal monitoring apparatus, or combinations thereof.

6. The device of any of claims 1-5 wherein said power supply is a rechargeable battery, a disposable battery, an electrical connection to a constant source of electrical power, or combinations thereof.

7. The device of any of claims 1-6 where said stimulation is electrical stimulation, a vibration transient, a sound transient, or combinations thereof.

8. The device of any of claims 1-7, further comprising one or more communication circuits for communicating and receiving data and/or external commands from one or more external devices, wherein said external devices are at least one mobile device, computer, computing device, cellular device, or combinations thereof, and wherein said communication circuit communicates with one or more external devices through Bluetooth, radio frequency, cellular, Wi-Fi, or combinations thereof.

9. The device of any of claims 1-8 further comprising a switching circuit for selectively using communication from the biological signal monitoring apparatus to adjust stimulation settings, wherein if said switching circuit allows for communication from said biological signal monitoring apparatus to said at least one controller, said at least one controller said stimulation circuit to adjust the stimulation intensity based on the biological state received by said biological signal monitoring apparatus of said user.

10. The device of any of claims 1-9 wherein said stimulation is an electrical stimulation, wherein said electrical stimulation is controlled by said controller by adjusting the electrical characteristics of the electrical stimulation, wherein said electrical characteristic is the amperage, pulse width, pulse frequency, duty cycle, waveform, or combinations thereof.

11. The device of claim 8 wherein said communication circuit allows for remote monitoring or remote programming of the electrical stimulation of said device.

## Patentansprüche

1. Tragbare Ohrvorrichtung (100) zum Abgeben einer Nervenstimulation im geschlossenen Kreislauf an eine Hautoberfläche, wobei die Vorrichtung umfasst:
ein oder mehrere Stimulationsübertragungsgeräte (140) zum Übertragen einer elektrischen Stimulation an die Hautoberfläche von mindestens einem Stimulationskreis aus;
mindestens einen Stimulationskreis (130) für die Erzeugung und Abgabe einer Stimulation to ein oder mehrere Stimulationsübertragungsgeräte, wobei der mindestens eine Stimulationskreis (130) mit dem einen oder den mehreren Stimulationsübertragungsgeräten elektrisch verbunden ist;
ein erstes Gerät (250) zum Überwachen von biologischen Signalen, wobei das erste Gerät (250) zum Überwachen von biologischen Signalen mindestens ein Detektor einer galvanischen Hautreaktion (GSR) zum Messen der Leitfähigkeit der Hautoberfläche ist;
einen oder mehrere Kommunikationskreise (360) zum Mitteilen und Empfangen von Daten und/oder externen Befehlen von einer oder mehreren externen Vorrichtungen an mindestens einen Regler, um auf Anforderung eine elektrische Stimulation von der einen oder den mehreren externen Vorrichtungen bereitzustellen;
mindestens einen Regler (120) in elektrischer Kommunikation mit dem mindestens einen Stimulationskreis (130), einem oder mehreren Kommunikationskreisen (360) und dem ersten Gerät (250) zum Überwachen von biologischen Signalen, wobei der Regler (120) dazu konfiguriert ist, dem Stimulationskreis (130) ein Stimulationssignal basierend auf der gemessenen Leitfähigkeit des Detektors der galvanischen Hautreaktion bereitzustellen;
wobei der Regler (120) dazu konfiguriert ist, dem Stimulationskreis (130) eine Stimulationsintensität auf einem Niveau von 50 % bis 90 % der Stimulationsintensität zwischen Parästhesie und einer vorbestimmten Stimulationsintensität, von der bekannt ist, das sie für einen Benutzer an der Hautoberfläche Unbehagen verursacht, bereitzustellen, wenn der GSR-Detektor einen Hautleitfähigkeit innerhalb eines vorbestimmten Leitfähigkeitsbereichs detektiert;
wobei die vorbestimmte Stimulationsintensität, von der bekannt ist, dass sie Unbehagen verursacht, für einen bestimmten Benutzer über eine anfängliche Kalibrierroutine vorkalibriert wird und in einem Speicher gespeichert wird, der für den Regler (120) zugänglich ist; und
wobei der Regler (120) die Stimulationsintensität in Echtzeit basierend auf Schwankungen bei den GSR-Ablesungen automatisch anpasst, so dass die Stimulation innerhalb eines benutzerspezifischen Wohlfühlfensters bleibt.

2. Vorrichtung nach Anspruch 1, ferner umfassend mindestens ein zweites Gerät zum Überwachen von biologischen Signalen, um einen biologischen Zustand eines Benutzers, der die Vorrichtung trägt, zu überwachen.

3. Vorrichtung nach Anspruch 2, wobei das mindestens eine zweite Gerät zum Überwachen von biologischen Signalen eine Puls-Plethysmographie-Vorrichtung, eine Photoplethysmographie- (PPG) Vorrichtung, eine Pulsoximeter- (PulseOx) Vorrichtung, eine galvanische Hautreaktions- (GSR) Vorrichtung, eine Elektroenzephalogramm- (EEG) Vorrichtung, eine Elektrokardiogramm- (ECG) Vorrichtung, eine Elektromyographie-(EMG) Vorrichtung oder eine Kombination derselben ist.

4. Vorrichtung nach einem der Ansprüche 1 bis 3, ferner umfassend mindestens ein Gehäuse, das eine äußere Oberfläche aufweist, die dazu gestaltet ist, in mindestens einen Teil eines menschlichen Ohrs eingesetzt zu werden, wobei das Gehäuse ein Innenvolumen definiert, und wobei das Gehäuse ein oder mehrere der Stimulationsübertragungsgeräte an seiner äußeren Oberfläche aufweist.

5. Vorrichtung nach Anspruch 4, wobei das Innenvolumen des Gehäuses eines oder mehrere von der Stromversorgung, dem Stimulationskreis, dem Stimulationsregler, dem ersten oder zweiten Gerät zum Überwachen von biologischen Signalen oder Kombinationen derselben enthält.

6. Vorrichtung nach einem der Ansprüche 1 bis 5, wobei die Stromversorgung eine wiederaufladbare Batterie, eine Einwegbatterie, eine elektrische Verbindung mit einer konstanten Quelle elektrischer Energie oder eine Kombination derselben ist.

7. Vorrichtung nach einem der Ansprüche 1 bis 6, wobei die Stimulation eine elektrische Stimulation, eine Vibrationstransiente, eine Tontransiente oder eine Kombination derselben ist.

8. Vorrichtung nach einem der Ansprüche 1 bis 7, ferner umfassend einen oder mehrere Kommunikationskreise zum Mitteilen und Empfangen von Daten und/oder externen Befehlen von einer oder mehreren externen Vorrichtungen, wobei die externen Vorrichtungen mindestens eine mobile Vorrichtung, ein Computer, eine Computervorrichtung, eine Mobilfunkvorrichtung oder Kombinationen derselben sind, und wobei der Kommunikationskreis mit einer oder mehreren externen Vorrichtungen über Bluetooth, Funkfrequenz, Mobilfunk, WiFi oder Kombinationen derselben kommuniziert.

9. Vorrichtung nach einem der Ansprüche 1 bis 8, ferner umfassend einen Schaltkreis, um eine Kommunikation von dem Gerät zum Überwachen von biologischen Signalen selektiv zu verwenden, um Stimulationseinstellungen anzupassen, wobei, falls der Schaltkreis eine Kommunikation von dem Gerät zum Überwachen von biologischen Signalen zu dem mindestens einen Regler ermöglicht, der mindestens eine Regler den Stimulationskreis [verwendet], um die Stimulationsintensität basierend auf dem biologischen Zustand, der von dem Gerät zum Überwachen von biologischen Signalen des Benutzers empfangen wird, anzupassen.

10. Vorrichtung nach einem der Ansprüche 1 bis 9, wobei die Stimulation eine elektrische Stimulation ist, wobei die elektrische Stimulation durch den Regler geregelt wird, indem die elektrischen Kennzeichen der elektrischen Stimulation angepasst werden, wobei die elektrischen Kennzeichen die Stromstärke, die Impulsbreite, die Impulsfrequenz, die Einschaltdauer, die Wellenform oder Kombinationen derselben sind.

11. Vorrichtung nach Anspruch 8, wobei der Kommunikationskreis eine entferne Überwachung oder eine entfernte Programmierung der elektrischen Stimulation der Vorrichtung ermöglicht.

## Revendications

1. Dispositif auditif portable (100) pour délivrer une stimulation nerveuse en boucle fermée sur une surface cutanée, le dispositif comprenant :
un ou plusieurs appareils de transfert de stimulation (140) pour transférer une stimulation électrique à ladite surface cutanée à partir d'au moins un circuit de stimulation ;
au moins un circuit de stimulation (130) pour la génération et la délivrance d'une stimulation à un ou plusieurs appareils de transfert de stimulation, ledit au moins un circuit de stimulation (130) étant électriquement connecté auxdits un ou plusieurs appareils de transfert de stimulation ;
un premier appareil de surveillance des signaux biologiques (250), ledit premier appareil de surveillance des signaux biologiques (250) étant au moins un détecteur de réponse cutanée galvanique (GSR) pour mesurer la conductance de la surface cutanée ;
un ou plusieurs circuits de communication (360) pour communiquer et recevoir des données et/ou des commandes externes provenant d'un ou plusieurs dispositifs externes vers au moins un contrôleur fournissant une stimulation électrique à la demande à partir desdits un ou plusieurs dispositifs externes ;
au moins un contrôleur (120) en communication électrique avec ledit au moins un circuit de stimulation (130), un ou plusieurs circuits de communication (360) et ledit premier appareil de surveillance des signaux biologiques (250), ledit contrôleur (120) étant configuré pour fournir un signal de stimulation au circuit de stimulation (130) sur la base de la conductance mesurée par le détecteur de réponse cutanée galvanique ;
dans lequel ledit contrôleur (120) est configuré pour fournir au circuit de stimulation (130) une intensité de stimulation comprise entre 50 % et 90 % de l'intensité de stimulation située entre la paresthésie et une intensité prédéterminée connue pour provoquer une gêne chez un utilisateur au niveau de ladite surface cutanée, lorsque ledit détecteur GSR détecte une conductance cutanée dans une plage de conductance prédéterminée ;
dans lequel ladite intensité de stimulation prédéterminée, connue pour provoquer une gêne, est pré-calibrée pour un utilisateur particulier via une procédure d'étalonnage initiale et mémorisée dans une mémoire accessible au contrôleur (120) ; et
dans lequel ledit contrôleur (120) ajuste automatiquement l'intensité de stimulation en temps réel sur la base des fluctuations des mesures de GSR, de sorte que la stimulation reste dans une plage de confort spécifique à l'utilisateur.

2. Dispositif selon la revendication 1, comprenant en outre au moins un deuxième appareil de surveillance des signaux biologiques pour surveiller un état biologique d'un utilisateur portant le dispositif.

3. Dispositif selon la revendication 2, dans lequel ledit deuxième appareil de surveillance des signaux biologiques est un pléthysmographe à impulsions, un photopléthysmographe (PPG), un oxymètre de pouls (PulseOx), un capteur de réponse cutanée galvanique (GSR), un électroencéphalographe (EEG), un électrocardiographe (ECG), un électromyographe (EMG) ou des combinaisons de ces derniers.

4. Dispositif selon une quelconque des revendications 1 à 3, comprenant en outre au moins un boîtier dont une surface extérieure est façonnée pour être insérée dans au moins une partie d'une oreille humaine, ledit boîtier définissant un volume intérieur et ledit boîtier comportant un ou plusieurs appareils de transfert de stimulation sur sa surface extérieure.

5. Dispositif selon la revendication 4, dans lequel ledit volume intérieur dudit boîtier contient un ou plusieurs éléments parmi l'alimentation électrique, le circuit de stimulation, le contrôleur de stimulation, le premier ou le deuxième appareil de surveillance des signaux biologiques ou des combinaisons de ces derniers.

6. Dispositif selon une quelconque des revendications 1 à 5, dans lequel l'alimentation électrique est assurée par une batterie rechargeable, une pile jetable, une connexion électrique à une source d'alimentation électrique continue ou une combinaison de ces dernières.

7. Dispositif selon une quelconque des revendications 1 à 6, dans lequel ladite stimulation est une stimulation électrique, une vibration transitoire, une stimulation sonore ou des combinaisons de ces dernières.

8. Dispositif selon une quelconque des revendications 1 à 7, comprenant en outre un ou plusieurs circuits de communication pour communiquer et recevoir des données et/ou des commandes externes provenant d'un ou plusieurs dispositifs externes, dans lequel lesdits dispositifs externes sont au moins un appareil mobile, un ordinateur, un dispositif informatique, un appareil cellulaire ou des combinaisons de ces derniers et dans lequel ledit circuit de communication communique avec un ou plusieurs dispositifs externes par Bluetooth, radiofréquence, réseau cellulaire, Wi-Fi ou des combinaisons de ces derniers.

9. Dispositif selon une quelconque des revendications 1 à 8, comprenant en outre un circuit de commutation pour utiliser sélectivement la communication provenant de l'appareil de surveillance des signaux biologiques pour ajuster les paramètres de stimulation, dans lequel si ledit circuit de commutation permet la communication entre ledit appareil de surveillance des signaux biologiques et au moins un contrôleur, ledit au moins un contrôleur ajuste ledit circuit de stimulation afin d'adapter l'intensité de stimulation sur la base de l'état biologique, tel que reçu par ledit appareil de surveillance des signaux biologiques dudit utilisateur.

10. Dispositif selon une quelconque des revendications 1 à 9, dans lequel ladite stimulation est une stimulation électrique, dans lequel ladite stimulation électrique est contrôlée par ledit contrôleur par ajustement des caractéristiques électriques de la stimulation électrique, dans lequel lesdites caractéristiques électriques sont l'ampérage, la largeur d'impulsion, la fréquence d'impulsion, le rapport cyclique, la forme d'onde ou des combinaisons de ces derniers.

11. Dispositif selon la revendication 8, dans lequel ledit circuit de communication permet la surveillance à distance ou la programmation à distance de la stimulation électrique dudit dispositif.
